# EUROPEAN PATENT APPLICATION

(11) **EP 4 252 722 A1**
(43) Date of publication of application: **04.10.2023**
(21) Application number: 22164817.3
(22) Date of filing: 28.03.2022
(51) Int. Cl.: A61F 5/01, A61F 5/058

(54) **WRIST BRACE**

(71) Applicant: Lang, Tobias, 9434 Au (CH)
(72) Inventor: Lang, Tobias, 9434 Au (CH)
(74) Representative: PPR AG

(57) **Abstract**

The invention provides a wrist brace (100) comprising a forearm part (200), configured to be strapped on or slipped in at a forearm of a person, a hand part (300), configured to be strapped on or slipped in at a hand of a person, and a bendable connector (400) connecting the forearm part and the hand part. The bendable connector (400) is removably engageable with both the forearm part and the hand part. To this end, the bendable connector (400) and the forearm part (200), respectively the hand part (300), comprise a joint construction (500), which is configured in such a way that the bendable connector is, preferably toolless, exchangeable, in particular from a set of different bendable connectors (400).

## Description

This invention relates to a wrist brace having a forearm part, a hand part, and a bendable attenuator, according to the pre-amble of claim 1.

Carpal tunnel syndrome, or median neuropathy at the wrist, is a medical condition in which the median nerve is compressed at the wrist leading to several medical phenomena, such as paraesthesia, numbness, and muscle weakness in the hand. Often these conditions are a result of frequent, sustained, and repetitive motion involving the hands of a patient, causing inflammation or strain of the bone, tendon or ligament apparatus.

Patients suffering from such conditions, or people prone to them, may find benefit from a wrist brace. In particular, extension of the hand, *i.e.* by bending the joint upwards and lifting the back of the hand towards the posterior forearm, can be painful. Flexion of the hand, bending it back-down to a neutral position or further down beyond a neutral position towards the anterior forearm, on the other hand very often is painless. Application of braces therefore intends to limit wrist extension or rotation, and to maintain the wrist in a neutral position. Over time, many braces have been developed for the treatment of wrist injuries. Most of the known braces are designed in the form of rigid cuffs which surround the arm in an approximately semicircular manner and fix the hand in an extended position. Moreover, DE8804120U1 discloses a wrist support which allows a certain freedom of movement of the supported hand. The wrist support disclosed consists of a forearm plate fixable to the forearm of a patient, a hand plate faxable to the patient's hand, and an integrally designed elastic connecting bar in the form of a leaf-spring connecting the forearm plate and the hand plate in an angular position relative to each other. In application, the hand plate functions as a cushion against further upward movement of the hand. The connecting bar may bend, providing a further reaction force limiting hand extension.

A disadvantage of the known wrist braces is that they do not provide an adjustability to specific patient requirements. In particular, they don't allow an option to adjust the attenuation force or resilience of the brace during the course of a patient's remedial therapy. Moreover, the known braces only limit an up-down extensionflexion motion of the hand and do not provide a remedial therapy for radial and ulnar deviation motion of the hand, nor for pronation-supination motion of the handforearm.

The inventors recognise the need of a wrist brace which provides an adjustability to the specific and changing requirements during the course of a patient's therapy or between different activities performed by the patient or user. In addition, the inventors recognise the need for a wrist brace providing remedial therapy possibilities for tendon, ligament, and muscles supporting the hands motion in multiple directions. The invention intends to alleviate at least one of the above-mentioned drawbacks of the prior art, respectively to provide a solution to the identified needs.

The objective of the invention is solved by the features of the independent claims. Advantageous further developments are shown in the figures and in the dependent claims.

According to an aspect of the invention, a wrist brace is provided comprising a forearm part, a hand part, and a connector connecting the forearm part and the hand part, wherein the connector is removably engageable with both the forearm part and the hand part. Preferably, the connector is bendable or flexible so that it may function as an absorber or attenuator. The connector and the forearm part, respectively the hand part, comprise a joint construction, which is configured in such a way that the connector is, preferably toolless, exchangeable, in particular with another connector from a set of different connectors each having different resilient characteristics. Advantageously, the modular design of the wrist brace allows exchanging the bendable connector for a version providing a larger or smaller attenuation of a hand's extension motion in accordance with the therapy needs of, or activities performed by, a patient or user. Thus, advantageously, adjusting the stiffness of the connector allows optimizing the therapy of a patient, respectively optimizes the support provided by the wrist brace to a user. The resilient deformation of the connector provides a counteracting force limiting the wrist movement. The modular design even allows exchanging the connector for a rigid component (unbendable or inflexible) to minimize the movement of a patient's wrist during an initial rehab phase.

In an embodiment of the wrist brace, the connector provides in use an attenuation force against flexing the hand of upto 6000 N, preferably between 1000 N and 5000 N, more preferably between 2000 N and 4000 N, and typically 2800 N.

In an embodiment of the wrist brace, the joint construction preferably comprises a rigid component and a flexible component arranged to be interlocking for providing the removable engageability. Preferably, the rigid component is a recess, and the flexible component is a key. Advantageously, such a configuration allows for the toolless exchangability of the connector. The key may be resiliently coupled to a constructive element of the wrist brace, e.g., the hand part, respectively the forearm part, and the recess formed in the connector. Alternatively, the key may be coupled to the connector and the recess may be formed in the hand / forearm part.

In an embodiment of the wrist brace, the connector comprises the rigid component, and forearm part, respectively the hand part, comprise the flexible component. In an alternative embodiment, connector comprises the flexible component, and the forearm part, respectively the hand part, comprises the rigid component. Advantageously, this allows to produce the connector economically and robustly, especially when the rigid part is comprised in the connector.

In an embodiment of the wrist brace, the forearm part and/or the hand part comprises an unlocking latch which is manually actuatable to release the joint construction. Advantageously, the latch ensures a secure connection by the joint construction and prevents a premature release. Thus, the latch advantageously ensures the supporting function of the wrist brace.

In an embodiment of the wrist brace, the connector is in the form of a leaf spring, preferably made of carbon fiber, fiberglass composite, aramid composite, polyamide composite, or mixtures thereof, in particular with a rectangular cross-section to be slid into a rectangular cross section groove in the forearm and/or hand part. Advantageously, the connector may be produced economically using a moulding or pressing process technology. Furthermore, the geometrical form factors (length, width, thickness) of the connector may be easily varied for manufacturing sets of connectors having different resilient characteristics. Moreover, using plastic materials, such a s the aramid and polyamide composites allows the forearm part and hand part to be economically produced using 3D-printing technology.

In an embodiment of the wrist brace, the connector has a first end for engagement with the forearm part, and a second end for engagement with the hand part, wherein the connector is constructed such that both ends are parallel, preferably the parallel ends lie in different parallel planes. Advantageously, this construction allows the connector to form an arc that spans the wrist and provide a support function even when the hand is in a neutral position.

In an embodiment of the wrist brace, the joint construction is arranged to allow rotational movements of a hand, preferably around at least one of a length direction of a forearm, and a direction quasi-perpendicular to the forearm. Advantageously, this provides the wearer more freedom to operate the hand while still being supported by the wrist brace. The term "quasi-perpendicular" in the context of the invention is to be understood as that the rotation axis around which the rotational motion of the hand is made lies within a 15° cone with the perpendicular to the length direction of the forearm at its centre. Preferably, the hand rotational movement allowed by the joint construction ranges form -15° to +15° left / right, and -60° to + 60° up / down relative to a neutral position of the hand extending in the length direction of the forearm.

In an embodiment of the wrist brace, the joint construction is arranged to provide a form-fit connection along a length direction (L) of the connector and a rotatable connection around a rotation axis (RA) quasi perpendicular to the length direction, and/or wherein rigid component has a first radius of curvature (R1) and the flexible component has a second radius of curvature (R2), wherein R1 > R2, preferably wherein the first radius of curvature (R1) is 1.05 to 1.25 times the second radius of curvature (R2). Advantageously, such a joint construction is especially functional in the hand part. For such a joint construction allows rotation of the hand in multiple directions, while at the same time preventing a movement of the connector in its length direction relative to the hand part. Alternative to the round/circular form of the recess and key described above, they may have ellipsoidal shapes providing a similar freedom of operation.

In an embodiment of the wrist brace, the joint construction of both the hand part and the forearm part has a joint construction as described previously. In an alternative embodiment, the joint construction of the forearm part is formed by (i) rigid joint component in the first end of the connector (for engagement with the forearm part) in the form of an elongated recess, and (ii) a flexible component in the forearm part. Preferably, the elongated recess has in the length direction (L) of the connector a length (L_{R}) between 5 mm and 25 mm. Preferably, the length (L_{R}) of the recess is between 2x and 5x the diameter (2xR2) of the flexible component. Advantageously, the elongated recess allows for a position-adjustment of the wrist brace to anatomy of the wearer.

In an embodiment of the wrist brace, the forearm part is configured with a base member configured to fit the forearm, and with an outside bridge configuration spanning in the forearm direction, in particular wherein aeration-holes in the base member are arranged under the bridge. Advantageously, this allows to manufacture a light forearm part while maintaining sufficient stiffness of the forearm part to redirect the attenuation force to the forearm.

In an embodiment of the wrist brace, at least one of the forearm part and the hand part comprises a plurality of aeration-holes for allowing aeration of underlying skin when the wrist brace is in use. Advantageously, this improves the wearing comfort and allows perspiration to be dispersed.

In an embodiment of the wrist brace, the forearm part and/or the hand part are configured with a textile surface on a side facing towards the person, in particular wherein the textile surface is configured with a foam material (or any other soft spacer material) covered by a cloth layer, preferably wherein the textile surface is mounted removable at the forearm part and/or the hand part. Advantageously, this improves the wearing comfort and allows removal for cleaning purposes.

In an embodiment of the wrist brace, the hand part is configured as a glove. Advantageously, this allows a user to conveniently slide the wrist brace onto the hand. The glove may in particular be a full-finger glove. Alternatively, it may be a half-finger glove, or a mixed martial art (MMA) glove.

According to another aspect, the invention provides a method for adapting a wrist brace, in particular a wrist brace according to any of embodiments described above, to a specific use case or purpose, the method providing the steps: (i) disengaging a first connector from a forearm part and from a hand part of the wrist brace by releasing a joint construction, which is configured in such a way that the connector is, preferably toolless, exchangeable; (ii) exchanging the first connector for a second connector having resilience characteristics different form the first connector; and (iii) assembling the wrist brace by engaging the second connector with the forearm part and the hand part, in particular wherein the resilience and/or geometry of the first and second connector is different and adapted to a specific use case or purpose of the wrist brace. Advantageously, the method allows adapting the wrist brace to different purposes, such as rehab therapy or sports like mountain biking or ice hockey.

Further advantages, features and details of the invention will be apparent from the following description, in which embodiments of the invention are described with reference to the drawings.

The list of reference signs as well as the technical content of the patent claims and figures are part of the disclosure. The figures are described coherently and comprehensively. Identical reference signs indicate identical components, reference signs with different indices indicate functionally identical or similar components.

The figures show:
- Fig. 1: a perspective view of a (disassembled) first embodiment of the wrist brace according to the invention;
- Fig. 2A: a top view of the first embodiment of the wrist brace according to the invention;
- Fig. 2B: a bottom view of the first embodiment of the wrist brace according to the invention;
- Fig. 3: a perspective view of a (assembled) first embodiment of the wrist brace according to the invention;
- Fig. 4A: a close-up of the hand part of the first embodiment of the wrist brace according to the invention;
- Fig. 4B: an exposed view of the interior of the hand part according to a first embodiment of the invention;
- Fig. 5: a close-up view of the bendable connector according to the first embodiment of the invention;
- Fig. 6: a close-up of the joint construction in an exposed view of the interior of the forearm part according to the first embodiment of the invention;
- Fig. 7: schematically the joint construction according to a first embodiment of the invention.

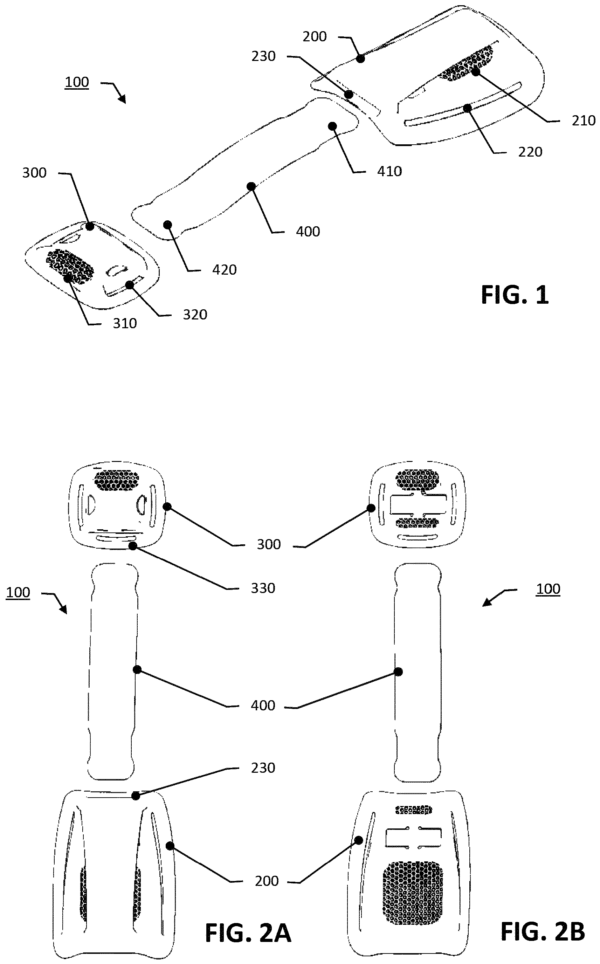

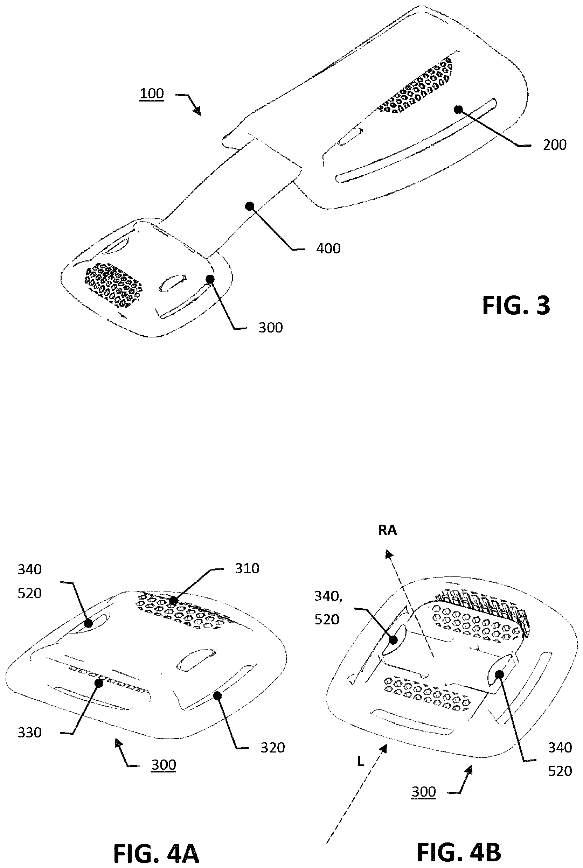

**Fig. 1** to **Fig. 3** schematically show an example of a wrist brace according to the invention. Shown is a first embodiment of a wrist brace 100 according to the invention in perspective view in a disassembled state (Fig. 1), respectively in an assembled state (Fig. 3). Fig. 2A shows a top view of the disassembled wrist brace 100 displaying the side of the wrist brace facing away from the arm and hand of a patient or user. Analogously, Fig. 2B show a bottom view displaying the side of the wrist brace facing towards the arm and hand.

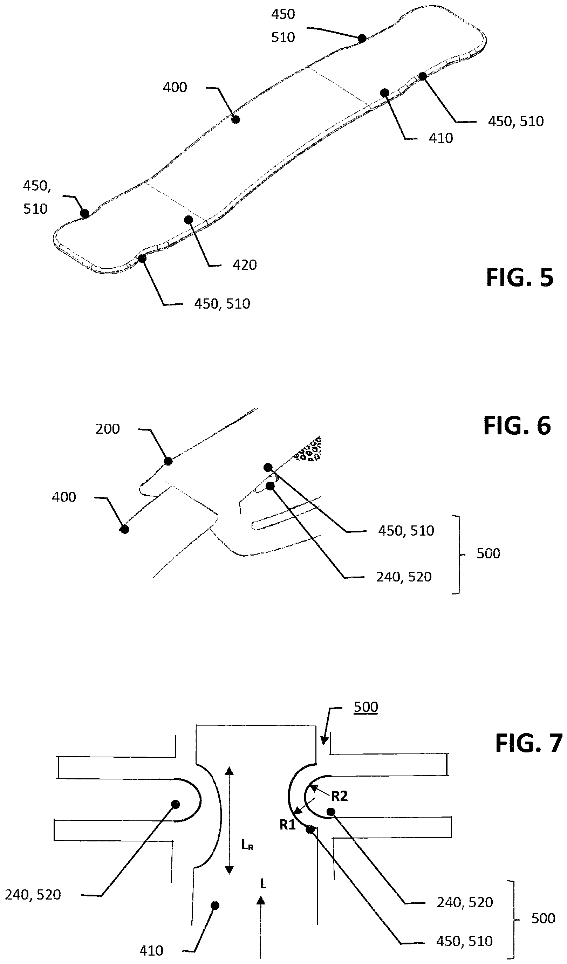

The wrist brace 100 comprises a forearm part 200, a hand part 300, and a connector 400. The connector preferably is resiliently bendable and has a first end 410 for engagement with the forearm part 200 and a second end 420 for engagement with the hand part 300 **(****Fig. 5**). For this purpose, both the forearm part 200 and the hand part 300 comprise a groove 230, 330 into which connector 400 may be removably inserted. For ventilation purposes of the - in use - underlying skin, forearm part 200 and/or hand part 300 may comprise aeration holes 210, 310. Forearm part 200 and hand part 300 may furthermore comprise fixation members 220, 320 allowing to fixate the wrist brace to the forearm, respectively the hand of the wearer. These fixation members may be formed as slots through which straps may be applied for fixating the wrist brace 100 to the forearm, respectively the hand. The straps may comprise buckle's, Velcro, or the like for conveniently fastening wrist brace 100. Alternatively, the fixation members may be arranged to allocate a quick release fastener employing a cable pull system. Alternatively still, the fixation member 320 of the hand part 300 may be provided in the form of a glove.

**Fig. 4** schematically shows a close-up of hand part 300. In **Fig. 4A** the exterior of hand part 300 is depicted, while **Fig. 4B** shows an exposed view of its interior. Depicted is groove 330 into which the second end 420 of resiliently bendable connector 400 may be inserted. To make a connection between hand part 300 and connector 400, a joint construction 500 is provided. Joint construction 500 is configured in such a way that bendable connector 400 is exchangeable. In particular, connector 400 may be selected from a set of different bendable connectors 400, each having specified stiffness properties. These specified stiffness properties can for instance be achieved through changes in the length, the width, or the thickness of the connector. Typically, the length ranges from 90 - 140 mm, the width from 26 - 30 mm, and the thickness from 1 - 3.5 mm. Preferably, exchanging the connector can be done toollessly. In an embodiment of the wrist brace, the joint construction is arranged to provide a form-fit connection along a length direction (L) of the connector and a rotatable connection around a rotation axis (RA) quasi perpendicular to the length direction.

Joint construction 500 comprises a rigid component 510 and a flexible component 520 arranged to be interlocking for providing the removable engageablity of connector 400 with hand part 300. As an example, the rigid component 510 may be a recess 450 provided in connector 400, and the flexible component 520 may be a key 340 provided in the hand part. Key 340 may be resiliently coupled to a constructive element of hand part 300.

In an alternative embodiment, the rigid component 510 may be a recess provided in hand part 300, while the flexible component 520 may be provided in connector 400.

Similar joint constructions 500 as described above, may be provided for making a connection between forearm part 200 and connector 400.

As an example, **Fig. 6** and **Fig. 7** show a close-up of joint construction 500 in the forearm part 200. As shown on the right-hand side of Fig. 7, rigid component 510 may have a first radius of curvature (R1) and the flexible component 520 may have a second radius of curvature (R2). Preferably, R1 > R2. Preferably the first radius of curvature (R1) is 1.05 to 1.25 times the second radius of curvature (R2). R1 may ranges from 6 to 8 mm, and typically is 7 mm, while R2 ranges from 5 to 7.5 mm, and typically is 5.9 mm.

As shown on the left-hand side of Fig. 7, ridged component 510 may be in the form of an elongated recess which has in the length direction (L) of the connector a length (LR) between 5 mm and 25 mm. Preferably, the length (LR) of the recess is between 2x and 5x the diameter (2xR2) of the flexible component.

As will be clear to the person skilled in the art, the embodiments and methods shown in the figures or described herein may also be combined and interchanged within the concept of the invention.

Advantageously, such combinations improve the adaptability of the wrist brace allowing adjustment in accordance with the therapy stage, the person preferences, or the specific requirements prescribed by the sport type. For performing sports like mountain biking and ice hockey often result in high and repetitive pressure loads on the wrist. Especially during intensive performance of these sports, and similar activities, the support of the wrist brace by redirecting the pressure load from the wrist to the forearm is valuable contribution to the safe and pain-free performance. Furthermore, with the fixation on the back of the hand, the wrist brace according to the invention ensures that the hands of the wearer are kept unobstructed. This allows the wearer free gripping of objects like the bicycle handlebars or the hockey stick.

### List of reference signs

- 100: wrist brace
- 200: forearm part
- 210: aeration holes of forearm part
- 220: slot for strap of forearm part
- 230: groove of forearm part
- 240: tongue / key
- 250: recess
- 300: hand part
- 310: aeration holes of hand part
- 320: slot for strap of hand part
- 330: groove of hand part
- 340: tongue / key
- 350: recess
- 400: bendable connector / attenuator
- 410: first end of bendable connector
- 420: second end of bendable connector
- 440: tongue / key
- 450: recess
- 500: joint construction
- 510: rigid joint component
- 520: flexible joint component
- L: Length direction of the bendable connector
- RA: Rotation Axis of joint construction
- R1: Radius of curvature of rigid joint component
- R2: Radius of curvature of flexible joint component

## Claims

1. A wrist brace (100) comprising
a forearm part (200), configured to be strapped on or slipped in at a forearm of a person,
a hand part (300), configured to be strapped on or slipped in at a hand of a person, and
a connector (400) connecting the forearm part and the hand part, **characterized in that**
the connector (400) is removably engageable with both the forearm part and the hand part, and the connector (400) and the forearm part (200),
respectively the hand part (300), comprise a joint construction (500), which is configured in such a way that the connector is, preferably toolless, exchangeable, in particular from a set of different bendable connectors (400).

2. The wrist brace (100) according to claim 1, wherein the joint construction (500) comprises a rigid component (510) and a flexible component (520) arranged to be interlocking for providing the removable engageability.

3. The wrist brace (100) according to claim 2, wherein the rigid component is a recess (250, 350, 450), and the flexible component is a key (240, 340, 440).

4. The wrist brace (100) according to any of claim 1 to 3, wherein the connector (400) comprises the rigid component (510), and forearm part (200), respectively the hand part (300), comprise the flexible component (520).

5. The wrist brace (100) according to any of claim 1 to 3, wherein connector (400) comprises the flexible component (520), and the forearm part (200), respectively the hand part (300), comprises the rigid component (510).

6. The wrist brace (100) according to any of claim 1 to 5, wherein the forearm part (200) and/or the hand part (300) comprises an unlocking latch which is manually actuatable to release the joint construction (500).

7. The wrist brace (100) according to any of claim 1 to 6, wherein the connector (400) is in the form of a leaf spring, preferably made of carbon fiber, fiberglass composite, aramid composite, polyamide composite, or mixtures thereof, in particular with a rectangular cross-section to be slid into a rectangular cross section groove (230, 330) in the forearm and/or hand part.

8. The wrist brace (100) according to claim 7, wherein the connector (400) has a first end (410) for engagement with the forearm part (200), and a second end (420) for engagement with the hand part (300), wherein the connector is constructed such that both ends are parallel, preferably the parallel ends lie in different parallel planes.

9. The wrist brace (100) according to any of claims 2 to 8, wherein the joint construction is arranged to allow rotational movements of a hand, preferably around at least one of a length direction of a forearm, and a direction perpendicular to the forearm.

10. The wrist brace (100) according to claim 9, wherein the joint construction (500) is arranged to provide a form-fit connection along a length direction (L) of the connector (400) and a rotatable connection around a rotation axis (RA) perpendicular to the length direction, and/or
wherein rigid component (510) has a first radius of curvature (R1) and the flexible component has a second radius of curvature (R2), wherein R1 > R2, preferably wherein the first radius of curvature (R1) is 1.05 to 1.25 times the second radius of curvature (R2).

11. The wrist brace (100) according to any of the claim 1 to 10, wherein the forearm part (200) is configured with a base member configured to fit the forearm, and with an outside bridge configuration spanning in the forearm direction, in particular wherein aeration-holes (210) in the base member are arranged under the bridge.

12. The wrist brace (100) according to any of the claims 1 to 11, wherein at least one of the forearm part (200) and the hand part (300) comprises a plurality of aeration-holes (210, 310) for allowing aeration of underlying skin when the wrist brace is in use.

13. The wrist brace (100) according to any of the claims 1 to 12, wherein the forearm part (200) and/or the hand part (300) are configured with a textile surface on a side facing towards the person, in particular wherein the textile surface is configured with a foam material covered by a cloth layer, preferably wherein the textile surface is mounted removable at the forearm part and/or the hand part.

14. The wrist brace (100) according to any of the claims 1 to 13, wherein the hand part (300) is configured as a glove.

15. A method for adapting a wrist brace (100), in particular a wrist brace (100) according to any of the claims 1 to 14, to a specific use case or purpose, of the wrist brace the method providing the steps
(i) disengaging a first connector (400) from a forearm part (200) and from a hand part (300) of the wrist brace (100) by releasing a joint construction (500), which is configured in such a way that the connector (400) is, preferably toolless, exchangeable;
(ii) exchanging the first connector (400) for a second connector (400) having resilience characteristics different form the first connector;
(iii) assembling the wrist brace (100) by engaging the second connector (400) with the forearm art (200) and the hand part (300), in particular wherein the resilience and/or geometry of the first and second connector is different and adapted to a specific use case or purpose of the wrist brace (100).
